# EUROPEAN PATENT SPECIFICATION

(11) **EP 2 197 385 B1**
(45) Date of publication and mention of the grant of the patent: **25.12.2013**
(21) Application number: 08847492.9
(22) Date of filing: 07.11.2008
(51) Int. Cl.: A61B 19/00

(54) **SURGICAL CONSOLE INFORMATION DISPLAY SYSTEM**
CHIRURGISCHE KONSOLE IN EINEM INFORMATIONSANZEIGESYSTEM
SYSTÈME D'AFFICHAGE DE DONNÉES D'UNE CONSOLE CHIRURGICALE

(30) Priority: 07.11.2007 US 986123 P
(43) Date of publication of application: 23.06.2010
(73) Proprietor: Alcon Research, Ltd., Fort Worth TX 76134 (US)
(72) Inventor: TEODORESCU, Dan, Fountain Valley California 92708 (US)
(74) Representative: Hanna, Peter William Derek
(86) International application number: PCT/US2008/082797
(87) International publication number: WO 2009/062031

(56) References cited:
- EP-A1- 1 704 839
- WO-A1-2007/030877
- US-A1- 2005 109 350
- US-A1- 2006 270 913
- US-A1- 2007 038 159
- US-A1- 2007 073 136

## Description

### BACKGROUND OF THE INVENTION

The human eye can suffer a number of maladies causing mild deterioration to complete loss of vision. While contact lenses and eyeglasses can compensate for some ailments, ophthalmic surgery is required for others. Generally, ophthalmic surgery is classified into posterior segment procedures, such as vitreoretinal surgery, and anterior segment procedures, such as cataract surgery, More recently, combined anterior and posterior segment procedures have been developed.

During a modem surgery, particularly ophthalmic surgery, a surgeon uses a variety of pneumatic and electronically driven microsurgical hand pieces. The hand pieces are operated by a microprocessor-driven surgical console that receives inputs from the surgeon or an assistant by a variety of peripheral devices, such as foot pedal controllers, infrared remote control devices and menu-driven touch screens. One such surgical console is described in U.S. Patent No. 5,455,766 (Scheller, et al.).

The setup and operation of an ophthalmic surgical console can be quite complex, as setting up a surgical instrumentation generally involves various electrical cables and pneumatic/fluidic tubing, etc. During setup and operation there are frequently periods during which an operator or surgeon is waiting, either for the surgical console to complete an action, such as a setup step, or for other reasons in the operating room, such as patient preparation, waiting for an assistant to complete a step or procedure, or due to other unanticipated reasons causing a delay in the surgical procedure at hand. In any such case, this time is idle time that could otherwise be used to instruct, inform or simply entertain the operator, surgeon and/or other persons in the general area of the surgical console. However, currently existing surgical consoles do not provide the ability to display much information beyond that directly related to the operation and use of the surgical console.

Therefore, there is a need for a surgical console information display system and method that can be used to provide information and content at a surgical console, beyond that provided by prior art surgical consoles, during appropriate times of a surgical procedure, surgical console setup or otherwise idle surgical console time.

EP 1,704,839-A and US 2007/073136-A are representative of the state of the art. EP 1,704,839-A discloses the features of the preamble of claim 1.

### SUMMARY OF THE INVENTION

The present invention provides a surgical console in accordance with claim 1. Preferred embodiments are disclosed in the dependent claims.

Embodiments of the present invention meet this need and others by providing a surgical console operable to provide audio and/or visual information at a surgical console display beyond that necessary for the operation and use of the surgical console. For example, the embodiments of the present invention can provide targeted advertisement, medical news and product updates, and current news and event information, among others. The embodiments of the present invention allow operators (e.g., surgeons), operating room personnel and others in the general vicinity of a surgical console to be informed, educated or simply entertained during, for example, idle time spent waiting on personnel or equipment during a surgical procedure, or simply during non-surgical use time of the surgical console. The embodiments of this invention can update or change the content and information provided by real-time connection over a wired or wireless network, such as wired or wireless Ethernet with a connection to the internet or a private data network, as will be known to those having skill in the art, or by a discrete upload of new content by, for example, scheduled software updates or content transfer from a peripheral device.

An embodiment of the present invention provides a surgical console used to facilitate intraocular surgery. This surgical console includes a processing module, memory device, user interface, and a network interface. The processing module is operable to direct the operations of an audio/visual display system and to receive inputs from a network or a peripheral device. These, peripheral devices include, for example, compact discs, hard drives, thumb-drives, DVDs, VHS tapes or any such storage device for storing and transferring audio/visual content.

Other advantages of the present invention will become more apparent to one skilled in the art upon reading and understanding the detailed description of the preferred embodiments described herein with reference to the following drawings.

### BRIEF DESCRIPTION OF THE DRAWINGS

For a more complete understanding of the present invention and the advantages thereof, reference is now made to the following description taken in conjunction with the accompanying drawings in which like reference numerals indicate like features and wherein:
FIG. 1 is a perspective view of one surgical console that may be used with embodiments of the present invention;
FIG. 2 is a functional block diagram of one surgical console in accordance with embodiments of the present invention;
FIGs. 3a-3c illustrate an example of the operation of an embodiment of the present invention during a surgical console start-up sequence;
FIGs. 4a-4c illustrate another example of the operation of an embodiment of the present invention during a surgical console 10 idle mode; and
FIG. 5 is a perspective view of a surgical console in accordance with embodiments of the present invention.

### DETAILED DESCRIPTION OF THE INVENTION

Preferred embodiments of the present invention are illustrated in the FIGs., like numerals being used to refer to like and corresponding parts of the various drawings.

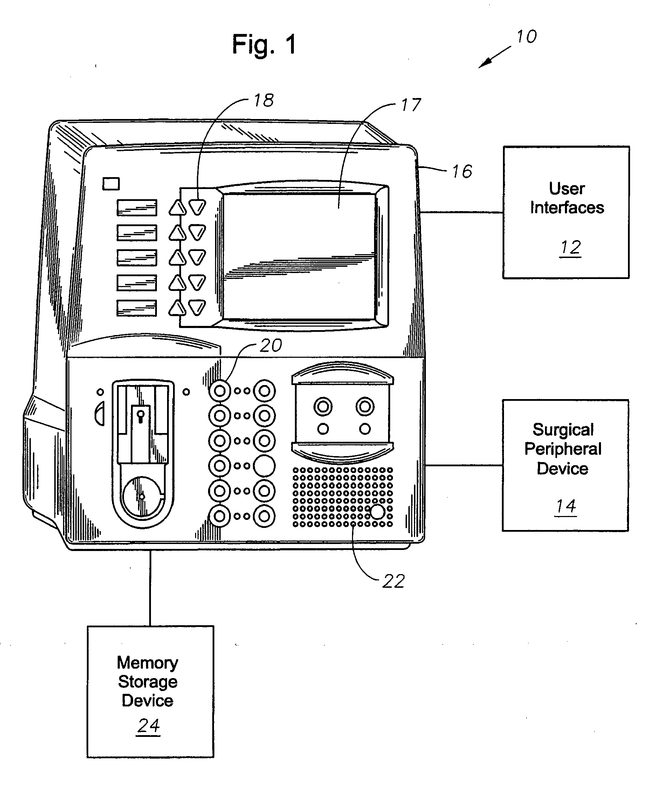

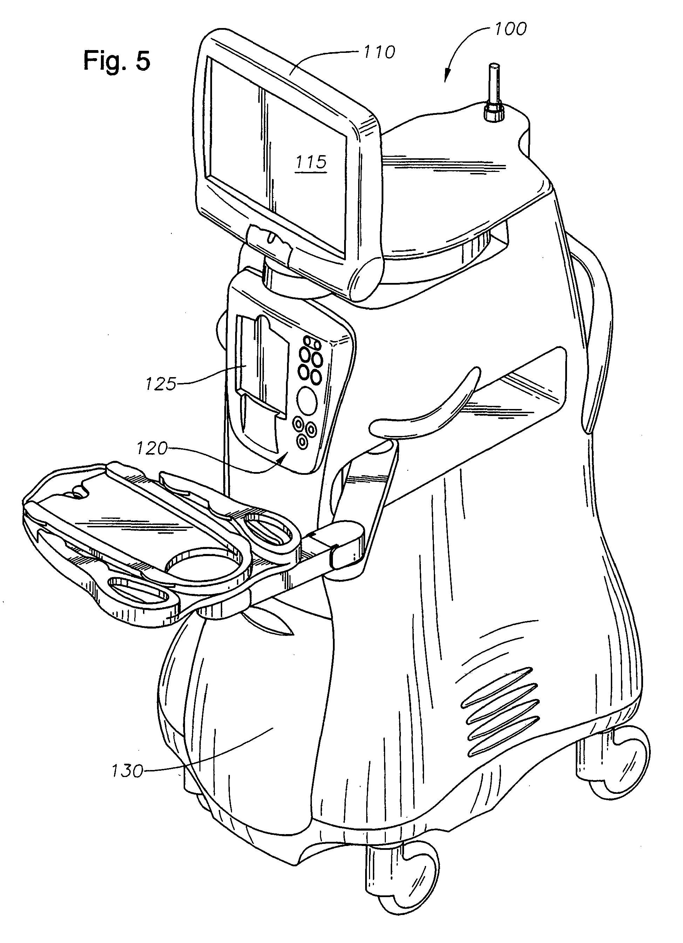

The surgical console information display system and method provided by embodiments of the present invention may be used with any suitable surgical console such as, but not limited to, the SERIES TWENTY THOUSAND® LEGACY®, the CONSTELLATION^{™}, INFINITI® or the ACCURUS® surgical system consoles, two of which are shown in FIG. 1 and FIG. 5, all commercially available from Alcon Laboratories, Inc., of Fort Worth, Texas

FIG. 1 provides an illustration of a surgical console 10 of one embodiment of the present invention. Surgical Console 10 may operably couple to a number of user interfaces 12, such as a foot pedal assembly or other push-button type assembly (not shown) and surgical peripheral devices 14. Console 10 allows an operator, such as a surgeon, to begin a surgical procedure by setting the initial operating parameters and modes into the console. This may be done by allowing the operator to interface with the surgical console through user interfaces 12 or other interfaces provided on the front panel 16. These interfaces may include an electronic display screen 17, one or more push-button switches or touch-sensitive pads 18, one or more endless digital potentiometer knobs 20, or other like interfaces known to those skilled in the art. Push-button switches 18 and knobs 20 are actuable by an operator to access various different operating modes and functions used in various surgical parameters. Console 10 may also include the ability to accept storage media such as cassette tapes, memory cards, floppy disks, compact discs (CDs), digital video disks (DVDs), or other like devices known to those skilled in the art.

Electronic display screen 17 may be controlled by a processing module that allows the operator access to one or more different menus or messages which relate to the functions and operations of the various push buttons 18 and knobs 20. In one embodiment the display screen 17 may be divided into display screen regions associated with individual buttons 18 or knobs 20. This arrangement allows for the indicated function of each button 18 or knob 20 to be readily changed. The use of the electronic display screen 17 also permits the buttons 18 and knobs 20 to be labeled in virtually any language. Electronic display screen 17 may be a touch screen, as will be known to those having skill in the art and may display representations of various surgical console 10 functions as described herein.

Surgical console 10 may be adapted for use with a number of different surgical instruments (i.e. surgical peripheral devices 14). For example, these may include a fiber optic illumination instrument, a surgical microscope, a vitrectomy unit, a fragmentation emulsification instrument, a cutting instrument, such as a guillotine cutter for vitrectomy procedures, and/or micro-scissors inset for proportionate and multiple cutting. While the above-identified microsurgical instruments are provided for illustrative purposes it should be understood that the surgical console 10 can be used with other similar equipped instruments. The surgical console 10 can also be attached to similar training devices that perform these same functions. In such a case, the surgical console 10 can then coordinate a training surgical procedure for the integrated use of the peripheral devices 14 or individual exercises (or games) that focus on specific piece(s) of equipment.

In general, any microsurgical instruments that are actuated or controlled by pneumatic or electronic signals may be operably coupled to and controlled by surgical console 10. This control or actuation may be governed by pneumatic, electronic, optical, or other like signals known to those skilled in the art wherein the signals are generated by surgical console 10. Each of these illustrated microsurgical peripheral devices 14 that couple to surgical console 10 may have different modes of operation that may require different settings or parameters that can be provided by the surgical console 10. By saving these operating parameters and surgical modes which are associated with specific steps of a surgical procedure in memory, the setup of the microsurgical peripheral devices 14 is facilitated by eliminating the often tedious or cumbersome process of initializing these devices manually via the surgical console 10 for each step of the surgical procedure.

Embodiments of the present invention can be used to provide information and content--audio, visual or both--to educate, inform or simply entertain users and others in the general vicinity of surgical console 10. For example, electronic display screen 17 can be used as an information "billboard", in one embodiment, to inform users and others of surgical products, such as ophthalmic surgical products, manufactured by the surgical console manufacturer (or other maker). Other content that can be displayed can include relevant medical news, other advertisements, general news, entertainment segments, product updates, music, movies, television shows, etc. Content can be provided as static images, flash animation, as audio/visual clips, or in any such audio and/or visual manner as will be known to those having skill in the art. Real-time news or other media feeds can be provided via, for example, a wired or wireless network interface connecting to the internet or another network.

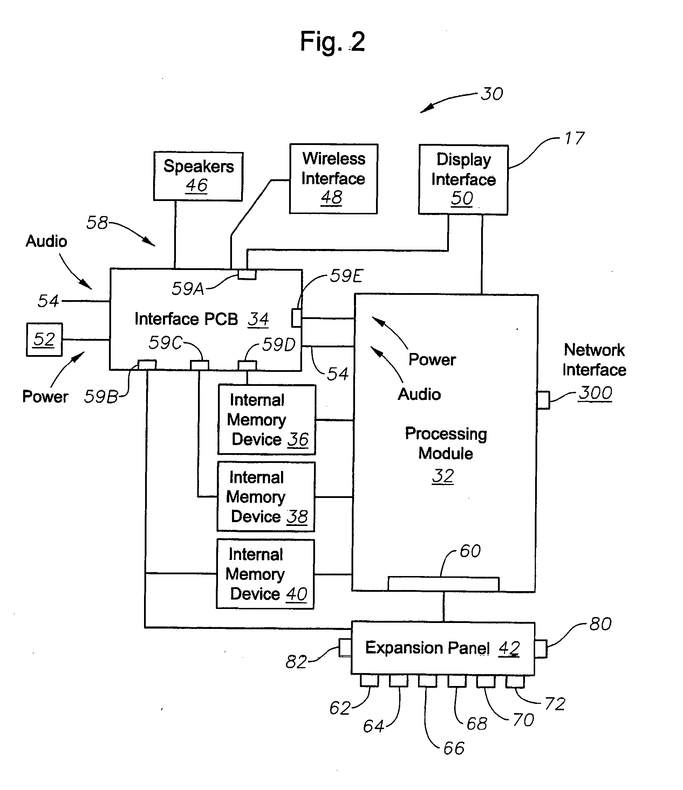

FIG. 2 is a simplified block diagram of various functional modules that may form part of surgical console 10. A surgical console 10 may functionally include a processing module 32, a power signal 52 provided to input-output (I/O) interface printed circuit board (PCB) 34, memory devices, which can be mass storage devices, 36, 38, and 40, audio output (speaker(s)) 46, display port or connectors 50, expansion panel 42, and an external connection to audio/video inputs. Interface PCB 34 may include an audio output 58, a power output 59, and audio input 54. Interface PCB 34 couples to an external or internal power supply 152, which provides power signal 52. Interface PCB 34 can distribute power to various other functional elements of surgical console 10. For example, power may be distributed through connections 59A, 59B, 59C, 59D and 59E to processing system 32, mass storage devices 36-40, expansion panels 42, and other functional units within the surgical console 10 as required. Additionally, interface PCB 34 may receive audio signals through audio/video inputs 54, either from an external source or from a connection to processing module 32. Interface PCB 34 can route audio inputs to audio output port 58 and speakers 46.

Mass storage devices 36-40 may comprise hard drives, DVD drives, CD drives, tape drives, VHS decks, solid-state memory and other like storage devices as will be known to those having skill in the art. Interface PCB 34 supplies power to mass storage devices 36-40. The content or other information contained within mass storage devices 36-40 may be accessed through various interfaces to processing module 32 and routed to an appropriate playback portion of the surgical console 10 by interface PCB 34. For example, an audio signal may be routed to a speaker 46 in the case of a digital audio file such as an MP3 file, wave file or other like file, or a video or image content can be provided to display module 17. Thus, mass storage devices 36-40, or an external multi-media playback device such as, but not limited to, an MP3 player, may be coupled to the PCB interface 34 to provide audio and/or video signals to Interface PCB 34 which may then be processed by processing module 32 and presented using an appropriate playback means, such as speakers 46 or display module 17. Additionally, control devices such as a keyboard or mouse may be coupled to interface PCB 34 to control the playback of multi-media files. Alternatively, in some embodiments buttons 18 and knobs 20, which may have functions defined as presented in display 17, may be used to control the playback of the multi-media content stored in mass storage devices 36-40 or on externally connected devices.

The processing module 32 may be a single processing device or a plurality of processing devices. Such a processing device may be a microprocessor, microcontroller, digital signal processor, microcomputer, central processing unit, field programmable gate array, programmable logic device, state machine, logic circuitry, analog circuitry, digital circuitry, and/or any device that manipulates signals (analog and/or digital) based on operational instructions. The memory may be a single memory device or a plurality of memory devices. Such a memory device may be a read-only memory, random access memory, volatile memory, non-volatile memory, static memory, dynamic memory, flash memory, cache memory, and/or any device that stores digital information. Note that when the processing module 32 implements one or more of its functions via a state machine, analog circuitry, digital circuitry, and/or logic circuitry, the memory storing the corresponding operational instructions may be embedded within, or external to, the circuitry comprising the state machine, analog circuitry, digital circuitry, and/or logic circuitry. The memory stores, and the processing module executes, operational instructions corresponding to at least some of the steps and/or functions illustrated in the FIGs. Processing module 32 includes a network interface 300 operable, as discussed above, to receive content in real-time or otherwise for display/playback at surgical console 10.

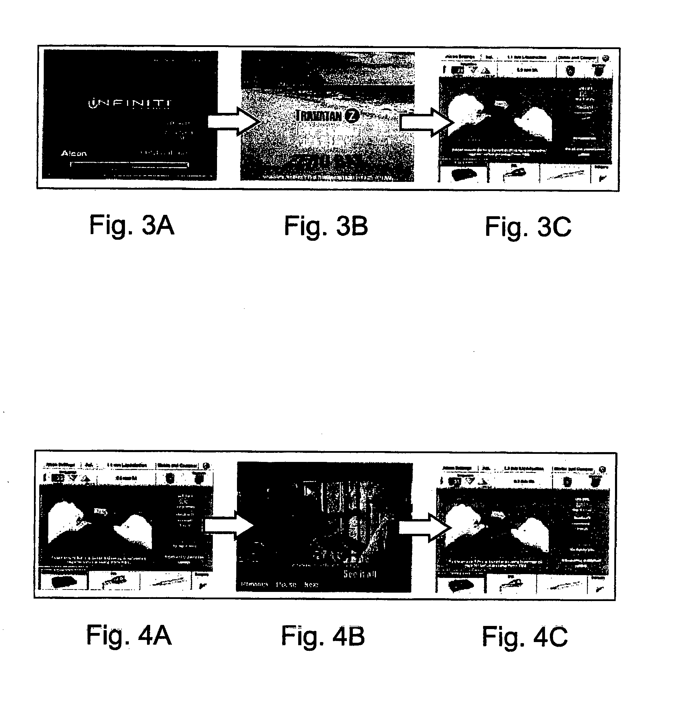

As can be seen from FIG. 2, surgical console 10 can be likened to a general purpose computer (processor) in that it can be programmed to perform various functions. The embodiments of the present invention take advantage of this functionality to display audio/visual information in much the same manner as a personal computer might, for example, during non-surgery modes of surgical console 10. Non-surgery modes can include power-up and set-up modes of surgical console 10, during which electronic display screen 17 can be used to display additional content as described herein. For example, during power-up of surgical console 10 a brief "splash screen", as known to those having skill in the art, can appear in between an application initialization screen and a set-up screen to display advertisements or other content. This example is illustrated in FIGs. 3a-3c. Once in the set-up screen, a feature similar to a screen saver can be used to display similar content during idle (non-surgery) console time. Non-surgery modes also can include any other time when the surgical console 10 is not being actively used for surgery, during a surgical procedure or otherwise.

FIGs. 4a-4c illustrate another example of the operation of an embodiment of the present invention during such surgical console 10 idle-mode times. As shown in FIG. 4a, surgical console 10 is waiting during a set-up screen, for example, while a user accomplishes the illustrated task. After an arbitrarily set period of time has elapsed, electronic display screen 17 may switch to display a selected audio and/or visual content, as shown in FIG. 4b. After a set time, or upon operator or surgical console 10 action to indicate a function has been completed, or that otherwise surgical console 10 is no longer idle, electronic display screen 17 will switch back, as shown in FIG. 4c, to a non-idle display.

Embodiments of the present invention can provide on-screen choices/functions for a user to control the information provided by surgical console 10 during such non-surgery modes. For example, as shown in FIG. 4b, "Previous", "Pause", "Next" and "Dismiss" functions can be selected by a user to change or pause the content provided during non-surgery modes. Further, a user/surgeon can select not to have any additional content displayed during non-surgery modes and can configure surgical console 10 such that operation of one or more connected peripheral devices, such as a footswitch or handpiece, will disable the display of non-surgical mode information.

FIG. 5 illustrates another surgical console 100 in which embodiments of the present invention may be implemented. It can be seen from FIG. 5 that a surgical console, such as surgical console 10 or 100, acts in cooperation with a number of consumables that require setup before a surgical procedure takes place. Embodiments of the present invention provide a system and method for making use of idle time associated with the setup and use of a surgical console 10 or 100.

FIG. 5 is a diagrammatic representation of one embodiment of an ophthalmic surgical console 100. Surgical console 100 can include a swivel monitor 110 that has touch screen 115. Swivel monitor 110 can be positioned in a variety of orientations for whomever needs to see touch screen 115. Swivel monitor 110 can swing from side to side, as well as rotate and tilt. Touch screen 115 provides a graphical user interface ("GUI") that allows a user to interact with console 100.

Surgical console 100 also includes a connection panel 120 used to connect various tools and consumables to surgical console 100. Connection panel 120 can include, for example, a coagulation connector, balanced salt solution receiver, connectors for various hand pieces and a fluid management system ("FMS") or cassette receiver 125. Surgical console 100 can also include a variety of user friendly features, such as a foot pedal control (e.g., stored behind panel 130) and other features.

In operation, a cassette (not shown) can be placed in cassette receiver 125. Clamps in surgical console 100 clamp the cassette in place to minimize movement of the cassette during use. The clamps can clamp the top and bottom of the cassette, the sides of the cassette or otherwise clamp the cassette. Surgical console 100 is provided by way of example and embodiments of the present invention can be implemented with a variety of surgical systems

Users of a surgical console in accordance with the embodiments of the present invention can be made aware or reminded of the product offerings of the surgical console manufacturer or of any other company, even when a sales representative is not on site. Further, users can be kept up to date in real-time via the network functionality of the embodiments of this invention. Any content created for "traditional" advertising or broadcasting media can be easily configured for display on a surgical console of the embodiments of this invention. Thus, news (e.g., breaking medical or other news), product offerings, entertainment or any such content can be provided to a user during otherwise idle time. In particular, surgical console advertising can be used to complement and supplement traditional forms of advertising, such as direct surgeon and staff interaction with a sales representative.

Descriptions of known programming techniques, computer software, hardware, operating platforms and protocols may be omitted so as not to unnecessarily obscure the invention in detail. It should be understood, however, that the detailed description and the specific examples, while indicating the preferred embodiments of the invention, are given by way of illustration only and not by way of limitation. Various substitutions, modifications, additions and/or rearrangements within the spirit and/or scope of the underlying inventive concept will become apparent to those skilled in the art from this disclosure.

As one of average skill in the art will appreciate, the term "substantially" or "approximately", as may be used herein, provides an industry-accepted tolerance to its corresponding term. Such an industry-accepted tolerance ranges from less than one percent to twenty percent and corresponds to, but is not limited to, component values, integrated circuit process variations, temperature variations, rise and fall times, and/or thermal noise. As one of average skill in the art will further appreciate, the term "operably coupled", as may be used herein, includes direct coupling and indirect coupling via another component, element, circuit, or module where, for indirect coupling, the intervening component, element, circuit, or module does not modify the information of a signal but may adjust its current level, voltage level, and/or power level. As one of average skill in the art will also appreciate, inferred coupling (i.e., where one element is coupled to another element by inference) includes direct and indirect coupling between two elements in the same manner as "operably coupled". As one of average skill in the art will further appreciate, the term "compares favorably", as may be used herein, indicates that a comparison between two or more elements, items, signals, etc., provides a desired relationship. For example, when the desired relationship is that signal 1 has a greater magnitude than signal 2, a favorable comparison may be achieved when the magnitude of signal 1 is greater than that of signal 2 or when the magnitude of signal 2 is less than that of signal 1.

Although the present invention is described in detail, it should be understood that various changes, substitutions and alterations can be made hereto without departing from the scope of the invention as described by the appended claims.

## Claims

1. A surgical console (10,100), comprising:
a processing module (32) operable to direct operations of and receive inputs from devices (14) operably coupled to the processing module;
at least one memory device (24) operably coupled to the processing module wherein the at least one memory device (24) is adapted to store multimedia content; and
a plurality of user interfaces (12) comprising a first user interface (17,100) and a second user interface (18,100);
**characterized in that**,
the first user interface (12) comprises an electronic display screen (17,110);
the second user interface (18,100) is disposed adjacent to the first user interface(17,100);
wherein the processing module is adapted to cause the first user interface (17,100) to display a representation of a surgical console function associated with the second user interface (18,100) when in a surgery mode of operation; and
wherein the processing module is adapted to cause the first user interface (17,100) to display selected portions of the multimedia content during idle console time or when in a non-surgery mode of operation.

2. The surgical console of claim 1, wherein the multimedia content comprises at least one of the group including advertisement, product updates, news, music, movies and television shows.

3. The surgical console of claim 1, further comprising a network interface (300) operable to provide multimedia content from a network for display on the user interface.

4. The surgical console of claim 3, wherein the network interface (300) is a wired or wireless Ethernet interface.

5. The surgical console of claim 3, wherein the network is the internet.

6. The surgical console of claim 1, wherein the memory device (24) is a mass storage device (36,38,40).

7. The surgical console of claim 6, wherein the mass storage device (36,38,40) is one of the group consisting of a hard disk drive, a DVD drive, a CD drive, a tape drive, a solid state memory, and a VHS deck.

8. The surgical console of claim 1, wherein the electronic display screen (110) is a touchscreen (115).

9. The surgical console of claim 1, wherein the non-surgery mode of operation is any time when the surgical console is not being used actively for surgery, during a surgical procedure or otherwise.

10. The surgical console of claim 1, wherein a non-surgery mode comprises a power-up mode, a set-up mode, or an idle time mode.

## Patentansprüche

1. Chirurgische Konsole (10, 100), aufweisend:
ein Prozessor-Modul (32), das zum Anweisen von Abläufen und zur Entgegennahme von Eingaben von Vorrichtungen (14) nutzbar ist, die an das Prozessor-Modul gekoppelt sind;
mindestens eine Speichervorrichtung (24), die an das Prozessor-Modul funktional gekoppelt ist, wobei die mindestens eine Speichervorrichtung (24) zum Speichern eines multimedialen Gehaltes vorgesehen ist; und
eine Mehrzahl an User-Interfaces (12), aufweisend ein erstes User-Interface (17, 100) und ein zweites User-Interface (18, 100);
**dadurch gekennzeichnet, dass**
das erste User-Interface (12) einen elektronischen Anzeigeschirm (17, 110) aufweist;
das zweite User-Interface (18, 100) angrenzend zu dem ersten User-Interface (17, 100) angeordnet ist;
wobei das Prozessor-Modul dazu bestimmt ist, das erste User-Interface (17, 100) zur Anzeige einer Darstellung einer Chirurgischen Konsolen-Funktion zu veranlassen, die mit dem zweiten User-Interface (118, 100) verknüpft ist, wenn ein betrieblicher chirurgischer Operationsmodus vorliegt; und
wobei das Prozessor-Modul dazu bestimmt ist, das erste User-Interface (17, 100) zur Anzeige ausgewählter Anteile des multimedialen Gehaltes während einer Stillstand-Zeit der Konsole oder im Falle eines betrieblichen chirurgischen Unterbrechungs-Modus zu veranlassen.

2. Chirurgische Konsole nach Anspruch 1, wobei der multimediale Gehalt mindestens einen Aspekt aus der Gruppe umfassend eine Anzeige, Produkt Updates, Neuigkeiten, Musik, Filme und Televisions-Shows umfasst.

3. Chirurgische Konsole nach Anspruch 1, ferner aufweisend ein Netzwerk-Interface (300), das zum Vorsehen eines multimedialen Gehalts aus einem Netzwerk zur Darstellung auf dem User-Interface betriebsfähig ist.

4. Chirurgische Konsole nach Anspruch 3, wobei das Netzwerk-Interface (300) ein kabelverbundenes oder kabelloses Ethernet-Interface ist.

5. Chirurgische Konsole nach Anspruch 3, wobei das Netzwerk das Internet ist.

6. Chirurgische Konsole nach Anspruch 1, wobei die Speichervorrichtung (24) eine Massenspeicher-Vorrichtung (36, 38, 40) ist.

7. Chirurgische Konsole nach Anspruch 6, wobei die Massespeicher-Vorrichtung (36, 38, 40) eine solche aus der Gruppe bestehend aus einer Hard-Disk-Drive einer DVD, einer Cd, eines Kasettenbandes, eines Solid-State-Speichers und eines VHS-Decks ist.

8. Chirurgische Konsole nach Anspruch 1, wobei der elektronische Anzeigeschirm (110) ein Touchscreen (115) ist.

9. Chirurgische Konsole nach Anspruch 1, wobei der betriebliche chirurgische Unterbrechungsmodus jeglichen Zeitabschnitt beinhaltet, wenn die chirurgische Konsole nicht aktiv für chirurgische Zwecke während eines chirurgischen Verfahrens in Verwendung ist, oder zu anderen Momenten.

10. Chirurgische Konsole nach Anspruch 1, wobei ein nicht chirurgischer Unterbrechungsmodus einen Power-Up Modus, einen Set-UP Modus oder einen Leerlauf-Zeitmodus umfasst.

## Revendications

1. Console chirurgicale (10, 100), comprenant :
un module de traitement (32) exploitable pour réaliser des opérations de, et recevoir des données provenant de dispositifs (14) couplés de manière opérationnelle au module de traitement ;
au moins un dispositif de mémoire (24) couplé de manière opérationnelle au module de traitement, dans lequel l'au moins un dispositif de mémoire (24) est adapté pour stocker du contenu multimédia ; et
une pluralité d'interfaces utilisateur (12) comprenant une première interface utilisateur (17, 100) et une deuxième interface utilisateur (18, 100) ;
**caractérisé en ce que**,
la première interface utilisateur (12) comprend un écran d'affichage électronique (17, 110) ;
la deuxième interface utilisateur (18, 100) est disposée de manière adjacente à la première interface utilisateur (17, 100) ;
dans lequel le module de traitement est adapté de telle manière que la première interface utilisateur (17, 100) affiche une représentation d'une fonction de console chirurgicale associée à la deuxième interface utilisateur (18, 100), lorsqu'il est en mode d'intervention chirurgicale ; et
dans lequel le module de traitement est adapté de telle manière que la première interface utilisateur (17, 100) affiche les parties sélectionnées du contenu multimédia au cours du temps d'inactivité de la console, ou lorsqu'il est en mode d'intervention non chirurgicale.

2. Console chirurgicale selon la revendication 1, dans laquelle le contenu multimédia comprend au moins un élément du groupe incluant des annonces publicitaires, des mises à jour de produits, des actualités, de la musique, des films et des émissions de télévision.

3. Console chirurgicale selon la revendication 1, comprenant en outre une interface réseau (300) exploitable de manière à fournir du contenu multimédia, provenant d'un réseau, destiné à être affiché sur l'interface utilisateur.

4. Console chirurgicale selon la revendication 3, dans laquelle l'interface de réseau (300) est une interface Ethernet câblé ou sans fil.

5. Console chirurgicale selon la revendication 3, dans laquelle le réseau est l'Internet.

6. Console chirurgicale selon la revendication 1, dans laquelle le dispositif de mémoire (24) est un dispositif de stockage de masse (36, 38, 40).

7. Console chirurgicale selon la revendication 6, dans laquelle le dispositif de stockage de masse (36, 38, 40) est un élément du groupe incluant un lecteur de disque dur, un lecteur de DVD, un lecteur de CD, un lecteur de bande, une mémoire à semiconducteurs et un lecteur VHS.

8. Console chirurgicale selon la revendication 1, dans laquelle l'écran d'affichage électronique (110) est un écran tactile (115).

9. Console chirurgicale selon la revendication 1, dans laquelle le mode d'intervention non chirurgicale est actif à tout moment lorsque la console chirurgicale n'est pas utilisée activement dans le cadre d'une intervention chirurgicale, pendant une procédure chirurgicale ou autre.

10. Console chirurgicale selon la revendication 1, dans laquelle un mode non chirurgical comprend un mode de mise sous tension, un mode de configuration, ou un mode de temps d'inactivité.
